# EUROPÄISCHE PATENTSCHRIFT

(11) **EP 0 657 142 B1**
(45) Veröffentlichungstag und Bekanntmachung des Hinweises auf die Patenterteilung: **11.04.2001**
(21) Anmeldenummer: 94118274.3
(22) Anmeldetag: 21.11.1994
(51) Int. Cl.: A61B 17/58

(54) **Knochenfixationselement**
Element for osteosynthesis
Elément d'ostéosynthèse

(30) Priorität: 07.12.1993 CH 364393
(43) Veröffentlichungstag der Anmeldung: 14.06.1995
(73) Patentinhaber: Synthes AG, Chur, 7002 Chur (CH)
(72) Erfinder: Schavan, Robert, CH-7260 Davos Dorf (CH); Frigg, Robert, CH-7270 Davos Platz (CH)
(74) Vertreter: Lusuardi, Werther Giovanni, Dr.

(56) Entgegenhaltungen:
- EP-A- 0 491 211
- DE-A- 3 418 490
- DE-A- 3 622 676
- DE-U- 8 631 649
- US-A- 2 388 482
- US-A- 2 532 296
- US-A- 4 537 185
- US-A- 5 098 435
- US-A- 5 334 204

## Beschreibung

Die Erfindung bezieht sich auf ein Knochenfixationselement gemäss dem Oberbegriff des Patentanspruchs 1, wie es aus der US-A-2 388 482 als bekannt hervorgeht.

Knochenschrauben und Drähte finden in der Osteosynthese in weiten Bereichen Anwendung. Knochenschrauben werden in der Osteosynthese sowohl zum Fixieren von Knochenfragmenten als auch zum Befestigen von Längsträgern eingesetzt. Drähte werden eingesetzt, um Knochenfragmente zu fixieren.

Konventionelle Knochenschrauben, insbesondere sogenannte Schanzsche Schrauben werden in der Osteosynthese für vielfältige Zwecke bei der Positionierung und Fixierung von Knochenfragmenten eingesetzt, beispielsweise als Bauelemente innerhalb einer osteosynthetischen Fixationsvorrichtung (sog. Fixateur externe, z.B. gemäss der EP-B1 0 153 546), welche im wesentlichen aus einem oder mehreren untereinander lösbar verbundenen Längsträgern und daran befestigbaren Klemmen oder Klammern zur Aufnahme der Schanzschen Schrauben besteht. Bei einer solchen Anwendung ist es notwendig vor dem Setzen der Schanzschen Schrauben ein entsprechendes Loch in den Knochen zu bohren, in welches dann die Schanzsche Schraube eingedreht werden kann. Dieser Vorgang ist kompliziert und zeitaufwendig.

Die Gewindesteigung einer Knochenschraube ist kritisch, da sie die Schnittgeschwindigkeit, d.h. den Vorschub der Schanzschen Schraube bestimmt, mit anderen Worten, die Schraube wird pro Umdrehung um die Höhe der Gewindesteigung nach vorne gezwungen. Wird eine, für Knochenschrauben übliche Gewindesteigung von ca. 1,75 mm verwendet, so muss die Bohrerspitze pro Umdrehung auf einer Strecke von 1,75 mm den Knochen durchbohren. Bei einer üblichen Umdrehungszahl einer in der Knochenchirurgie verwendeten Bohrmaschine von ca. 600 - 800 U/min entspricht dies einem Vorschub der Schraube von über einem Meter pro Minute. Der durch diesen Vorschub bedingte, ausserordentlich grosse Druck der Bohrerspitze auf den Knochen, kann Spontanrisse oder Frakturen im Knochen bewirken. Der Hauptnachteil eines zu grossen Schraubenvorschubes zeigt sich vor allem dann, wenn die Bohrerspitze die nahe Kortikalis und den Markraum durchdrungen hat und auf die Innenseite der Gegenkortikalis trifft. Dort hat die Bohrerspitze keine Gelegenheit, sich zu zentrieren und den sogenannten Anschnitt zu tätigen. Statt den Knochen zu durchbohren, wird dieser durch die Schraubenspitze weggestossen. Dies führt unweigerlich zu einem Bersten der Gegenkortikalis. Wenn die Schraube nahe der Fraktur eingebracht wird, entlädt sich der aufgebrachte Druck durch Längsspaltung der Gegenkortikalis.
Der Versuch den axialen Druck durch Reduktion der Bohrmaschinendrehzahl zu verkleinern ist nicht möglich, da der Vorschub durch die Gewindesteigung der Schraube vorgegeben ist. Die Arbeitsvorgänge zum Einbringen der Knochenschrauben sind aber auch umfangreich. Das Schraubenloch muss zuerst mit einem ersten Instrument aufgebohrt werden, dann muss mit einem zweiten Instrument das Gewinde geschnitten werden und schliesslich kann in einem dritten Arbeitvorgang die Knochenschraube eingedreht werden. Auch werden übliche Knochenschrauben nicht selten bis an die Grenze ihrer Haltekraft angezogen, um Längsträger, bzw. Knochenplatten auf dem Knochen zu fixieren. Dies ist nötig, da die Knochenschrauben mit Längsträgern keine stabile Verbindung eingehen können. Durch die oft sehr hohe Vorspannung bis an die Grenze der Haltekraft kann es vorkommen, dass die Schrauben aus dem Knochen ausreissen. Während der oben beschriebenen Arbeitvorgänge können sich zudem Fehler häufen und es kommt zu einer schlechten Fixation.
Drähte können zwar ohne grössere Vorarbeiten in den Knochen eingedreht werden, schädigen den Knochen aber durch die dabei auftretenden hohen Temperaturen, welche durch die Reibung zwischen Knochen und Draht entstehen. Des weiteren ist die axiale Stabilität von mit Drähten fixierten Knochenfragmenten nicht sehr hoch, so das es zum Verlust der Reposition kommen kann.
Aus der US-A-2388382 HAYNES ist ein Knochenfixationselement bekannt mit einem Kopfende, einem mindestens teilweise mit einem Gewinde versehenen Schaftteil und einem relativ zur Länge des Knochenfixationselementes kurzen Vorderteil mit Spitze, wobei der Vorderteil als Bohrer ausgebildet ist.

Hier will die Erfindung Abhilfe schaffen. Der Erfindung liegt die Aufgabe zugrunde, ein Fixationselement zu schaffen, welches ohne Vorarbeiten in den Knochen eingebracht werden kann.

Die Erfindung löst die gestellte Aufgabe mit einem Knochenfixationselement, welches die Merkmale des Anspruchs 1 aufweist. Weitere vorteilhafte Ausgestaltungen der Erfindung sind in den abhängigen Ansprüchen gekennzeichnet.

Das als zweilippiger Bohrer ausgebildete Vorderteil (Länge: 4 bis 12 mm / Gewindehöhe 0,6 mm bis 0,8 mm) des Knochenfixationselementes ist zweckmässigerweise als selbstschneidender, selbstbohrender Spiralbohrer ausgebildet, dessen Aussendurchmesser höchstens so gross ist als der Gewindekerndurchmesser.
Für den als Bohrer ausgebildeten Vorderteil sollte vorteilhafterweise die nachstehende Geometrie eingehalten werden.

| | |
|---|---|
| Spitzenwinkel | 60° bis 120°, vorzugsweise 80 - 90°; |
| Steigungswinkel | 10° - 40°, vorzugsweise 20 - 30°; |
| Fasenbreite | 0,2 mm - 1,0 mm, vorzugsweise 0,3 mm - 0,6 mm; |
| Bohrerseele | das 0,1 - 0,6-fache, vorzugsweise das 0,3 bis 0,5-fache des Bohrerdurchmessers in Abhängigkeit des Werkstoffs. |

Bezüglich der Bohrer-Terminologie wird auf das Werk "Metallbearbeitung", Band 1, Seite 146 (Verlag Otto Walter AG, Olten, 1950) verwiesen.

Die Erfindung und Weiterbildungen der Erfindung werden im folgenden anhand der teilweise schematischen Darstellungen eines Ausführungsbeispiels noch näher erläutert.

Es zeigen:
Fig. 1 eine schematische Darstellung des erfindungsgemässen Knochenfixationselementes;
Fig. 2 eine axiale Aufsicht auf das Knochenfixationselement nach Fig. 1;
Fig. 3 einen Detail-Längsschnitt durch das Gewinde des Knochenfixationselementes nach Fig. 1; und
Fig. 4 einen Schnitt längs der Linie A-A von Fig. 1.

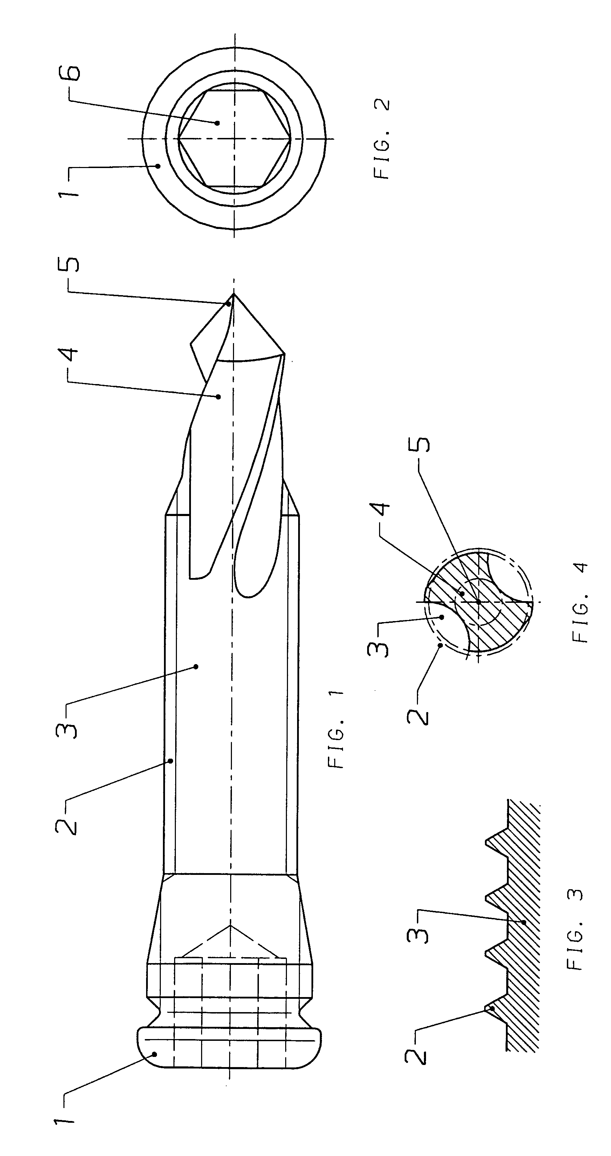

Das in den Fig. 1 - 4 dargestellte Knochenfixationselement besteht im wesentlichen aus einem Kopfende 1, einem mit einem Gewinde 2 versehenen Schaftteil 3 und einem Vorderteil 4 mit Spitze 5.
Der Vorderteil 4 ist als selbstschneidender, selbstbohrender, zweilippiger Spiralbohrer ausgebildet, dessen Aussendurchmesser kleiner ist als der Kerndurchmesser des Gewindes 2, welches sich bis in den Vorderteil 4 hinein erstreckt. Das Vorderteil 4 weist je nach Gesamtlänge des Knochenfixationselementes eine Länge von 7 - 9 mm auf.
Das Kopfende 1 ist - wie in Fig. 2 dargestellt - mit einer zentralen Vertiefung 6, in Form eines Innensechskantes, versehen, welche zur Aufnahme eines Eindrehinstrumentes dient.

Das Knochenfixationselement kann vielfältig verwendet werden, z.B. zur Fixation von Knochenplatten oder als Befestigungskomponente für Fixateurs externes in der Osteosynthese.

## Patentansprüche

1. Knochenfixationselement mit einem Kopfende (1), einem mindestens teilweise mit einem Gewinde (2) versehenen Schaftteil (3) und einem Vorderteil (4) mit Spitze (5), wobei der Vorderteil (4) als zweilippiger Bohrer ausgebildet ist,
**dadurch gekennzeichnet, dass**
A) der Vorderteil (4) eine Länge von 4 bis 12 mm aufweist; und
B) der als Bohrer ausgebildete Vorderteil (4) eine Gewindehöhe im Bereich von 0,6 - 0,8 mm aufweist.

2. Knochenfixationselement nach Anspruch 1, dadurch gekennzeichnet, dass der als Bohrer ausgebildete Vorderteil (4) selbstbohrend und selbstschneidend ist.

3. Knochenfixationselement nach Anspruch 1 oder 2, dadurch gekennzeichnet, dass der als Bohrer ausgebildete Vorderteil (4) einen maximalen Aussendurchmesser aufweist, welcher höchstens so gross ist als der Kerndurchmesser des Gewindes (2).

4. Knochenfixationselement nach einem der Ansprüche 1 - 3, dadurch gekennzeichnet, dass der Vorderteil (4) als Spiralbohrer ausgebildet ist.

5. Knochenfixationselement nach einem der Ansprüche 1 bis 4, dadurch gekennzeichnet, dass sich das Gewinde (2) bis in den Vorderteil (4) hinein erstreckt.

6. Knochenfixationselement nach einem der Ansprüche 1 bis 5, dadurch gekennzeichnet, dass das Kopfende (1) mit einer zentralen Vertiefung (6) zur Aufnahme eines Eindrehinstrumentes versehen ist.

7. Knochenfixationselement nach Anspruch 6, dadurch gekennzeichnet, dass die zentrale Vertiefung (6) ein Innensechskant ist.

8. Knochenplatte mit Knochenfixationselementen nach einem der Ansprüche 1 bis 7.

9. Fixateur externe mit Knochenfixationselementen nach einem der Ansprüche 1 bis 7.

## Claims

1. Bone fixation element having a head end (1), a shaft section (3) which is at least partially provided with a thread (2) and a front section (4) with tip (5), whereby the front part (4) is configured as a double-lipped drill,
characterised in that
A) the front section (4) has a length from 4 to 12 mm, and
B) the front section (4) shaped as a drill has a height of the thread in the range between 0,6 to 0,8 mm.

2. Bone fixation element according to claim 1, wherein the front section (4) that is configured as a drill is self-drilling and self-cutting.

3. Bone fixation element according to claim 1 or 2, wherein the front section (4) that is configured as a drill has a maximum outer diameter, which is at most as large as the core diameter of the thread (2).

4. Bone fixation element according to one of the claims 1 to 3, wherein the front section (3) is shaped as a spiral drill.

5. Bone fixation element according to one of the claims 1 to 4, characterised in that the thread (2) extends until into the front section (4).

6. Bone fixation element according to one of the claims 1 to 5, characterised in that the head end (1) is provided with a central indentation (6) for admitting an instrument for screwing the element into a bone.

7. Bone fixation element according to claim 6, wherein the central indentation (6) is a hexagonal socket.

8. Bone plate comprising bone fixation elements according to one of the claims 1 to 7.

9. External Fixation apparatus comprising bone fixation elements according to one of the claims 1 to 7.

## Revendications

1. Elément d'ostéosynthèse doté d'une extrémité de tête (1), d'une partie de tige (3) dont au moins une partie est dotée d'un filet (2) et d'une partie avant (4) dotée d'une pointe (5), la partie avant (4) étant configurée comme mèche à deux lèvres, caractérisé en ce que
A) la partie avant (4) présente une longueur de 4 à 12 mm ; et
B) la partie avant (4) configurée comme mèche présente un filet dont le pas est compris entre 0,6 et 0,8 mm.

2. Elément d'ostéosynthèse selon la revendication 1, caractérisé en ce que la partie avant (4) configurée comme mèche est autotaraudeuse et autoforante.

3. Elément d'ostéosynthèse selon la revendication 1 ou 2, caractérisé en ce que la partie avant (4) configurée comme mèche présente un diamètre extérieur maximum qui est au plus aussi grand que le diamètre de l'âme du filet (2).

4. Elément d'ostéosynthèse selon l'une des revendications 1 à 3, caractérisé en ce que la partie avant (4) est configurée comme mèche spiralée.

5. Elément d'ostéosynthèse selon l'une des revendications 1 à 4, caractérisé en ce que le filet (2) s'étend jusqu'à l'intérieur de la partie avant (4).

6. Elément d'ostéosynthèse selon l'une des revendications 1 à 5, caractérisé en ce que l'extrémité de tête (1) est dotée d'un creux central (6) destiné à recevoir un instrument de vissage.

7. Elément d'ostéosynthèse selon la revendication 6, caractérisé en ce que le creux central (6) est un hexagone intérieur.

8. Plaque d'ostéosynthèse dotée d'éléments d'ostéosynthèse selon l'une des revendications 1 à 7.

9. Fixateur externe doté d'éléments d'ostéosynthèse selon l'une des revendications 1 à 7.
